# EUROPEAN PATENT SPECIFICATION

(11) **EP 2 759 016 B1**
(45) Date of publication and mention of the grant of the patent: **29.03.2017**
(21) Application number: 12833458.8
(22) Date of filing: 23.09.2012
(51) Int. Cl.: H01M 8/18

(54) **ADDITIVES FOR HYDROGEN/ BROMINE CELLS**
ADDITIVE FÜR WASSERSTOFF-/BROMZELLEN
ADDITIFS POUR CELLULES HYDROGÈNE/BROME

(30) Priority: 22.09.2011 US 201161537622 P
(43) Date of publication of application: 30.07.2014
(73) Proprietor: Bromine Compounds Ltd., Beer-Sheva 84101 (IL)
(72) Inventor: MAGNES, Ben-Zion, 85025 Meitar (IL); LANCRY, Eli, 77407 Ashdod (IL); BERGSTEIN - FREIBERG, Mira, 84965 Omer (IL)
(74) Representative: Ter Meer Steinmeister & Partner
(86) International application number: PCT/IL2012/000349
(87) International publication number: WO 2013/042110

(56) References cited:
- EP-A1- 0 404 188
- US-A- 4 065 601
- US-A- 4 065 601
- US-A- 4 631 240
- NISHIDA T ET AL: "Physical and electrochemical properties of 1-alkyl-3-methylimidazolium tetrafluoroborate for electrolyte", JOURNAL OF FLUORINE CHEMISTRY, ELSEVIER, NL, vol. 120, no. 2, 1 April 2003 (2003-04-01) , pages 135-141, XP004413662, ISSN: 0022-1139, DOI: 10.1016/S0022-1139(02)00322-6

## Description

The invention relates to compounds suitable as additives for an electrolyte used in hydrogen/bromine cells, for complexing the elemental bromine formed in such cells.

There exists a need, in electrochemical flow cells which involve the generation of elemental bromine, to keep the bromine in a form which can be readily stored and pumped over a broad temperature range, such that it can be used without interfering with the operation of the flow cell.

The hydrogen/bromine cell is an example of a regenerative fuel cell. The operation of hydrogen/ bromine regenerative fuel cells is based on the electrolysis of hydrogen bromide, and the conversion of the electrolysis products, i.e., hydrogen and elemental bromine, back to hydrogen bromide. During charge, an electric current supplied from an external source drives the electrolysis of hydrogen bromide, generating hydrogen (H₂) and elemental bromine (Br₂), which are stored separately in suitable tanks located externally to the cell. H₂ and Br₂ are fed back to the cell during discharge and are reacted to give hydrogen bromide, thereby producing electric energy.

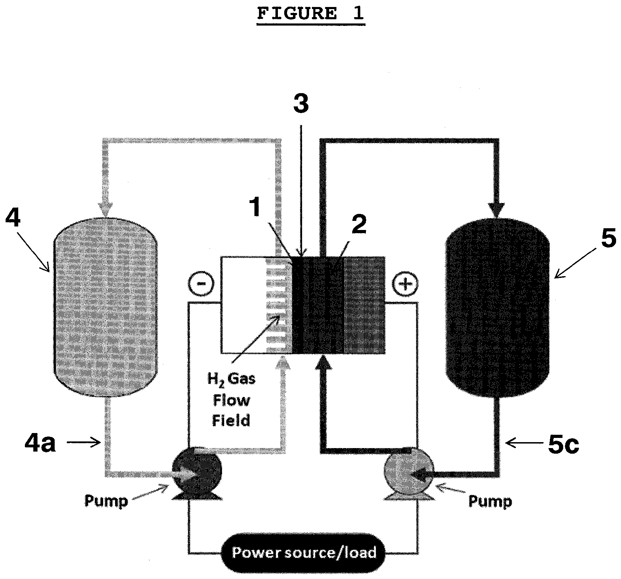

A characteristic hydrogen/bromine cell is shown diagrammatically in Figure 1. While this figure depicts a single cell, it is to be noted that a plurality of such cells can be assembled in series. Numerals **1** and **2** represent the hydrogen and bromine electrodes, respectively, and numeral **3** represents a separator (e.g. an ion exchange membrane) positioned between the electrodes. The term "hydrogen electrode" is used herein to indicate the electrode where hydrogen gas is formed (during charge) and oxidized (during discharge). The term "bromine electrode" is used herein to indicate the electrode where elemental bromine is formed (during charge) and reduced (during discharge).

A first storage tank, for collecting the hydrogen gas, is indicated by numeral **4.** A second storage tank, which contains a concentrated aqueous solution of hydrogen bromide, is indicated by numeral **5.** Flow paths **4a** and **5c,** connecting the hydrogen storage tank **4** and the HBr storage tank **5** to the respective sides of the cell, and pumps for driving the fluids along the flow paths are also shown in Figure 1.

The charge/discharge cycle is represented by the following pair of chemical equations:

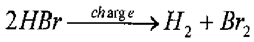

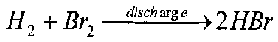

During charge, the electrolyte which comprises hydrobromic acid is fed from storage tank **5** to that side of the cell where the bromine electrode **2** is placed (which is the cathodic side of the cell at the charge state). The hydrobromic acid undergoes electrolysis, resulting in the formation of elemental bromine at the cathode. The electrolyte, enriched with elemental bromine, is removed from the cathodic side of the cell and is transferred to the storage tank **5**. Hydrogen ions concurrently pass across the membrane **3** to the anodic side, where hydrogen gas evolves at the anode **1,** and is collected in tank **4.**

During discharge, the hydrogen gas and the bromine-containing electrolyte are fed from their storage tanks **4** and **5,** respectively, to the respective sides of the cell, where the hydrogen and bromine electrodes are positioned (it is to be noted that the anodic/cathodic sides are reversed relative to previous stage). The reaction between hydrogen and bromine yields hydrobromic acid, with electric current being drawn from the cell.

It should be understood that the electrolyte, with which the electrolysis stage starts, is not necessarily free of elemental bromine. In practice, the electrolysis stage starts with an electrolyte which contains, in addition to hydrobromic acid, also up to 10% elemental bromine. For example, the concentrations of HBr and Br₂ in the aqueous electrolyte prior to the electrolysis may be from 5% to 52% (more preferably 10% to 45%) and from 0% to 10% (more preferably 0.1% to 5%), respectively. During the electrolysis stage (i.e., the charging process), the concentration of the hydrobromic acid in the electrolyte is gradually decreased, while the concentration of the elemental bromine increases. Upon completion of the charge state, the electrolyte typically comprises from 5% to 35% HBr and from 0.2M to 3.5M Br₂. It follows that the composition of the electrolyte varies significantly during the charge/discharge cycle.

Bromine is a dark red, fuming liquid. It is reactive and corrosive and has a high vapor pressure at room temperature. In cells utilizing bromine as an electrochemically active element, there is a need to deactivate the bromine, i.e., convert it into a form with reduced vapor pressure, which form is less likely to interfere with the operation of the cell. It is known in the art that this goal can be achieved by adding a bromine-complexing agent to the electrolyte. The bromine-complexing agent combines with bromine molecule(s) to form a polybromide complex. As a result, the vapor pressure above the complexed bromine solution is decreased.

In regenerative fuel cells the electrolyte reservoir is separated from the electrodes stack, with the electrolyte being pumped from the reservoir to the electrodes and back. The flowability of the electrolyte must be maintained with respect to different compositions corresponding to different states of charge, and over the entire operational temperature range (typically between -15°C and 50°C). In other words, throughout the operation of the cell, the formation of a solid phase in the electrolyte is unacceptable.

In the past, bromine complexing agents were investigated for the deactivation of elemental bromine in zinc bromine flow batteries. Bromine deactivation in these batteries may be achieved by the use of cyclic quaternary ammonium bromides (abbreviated quats) as complexing agents. In their most general form, these salts are represented by the following formula: 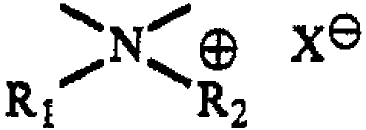 where R₁ and R₂ indicate the alkyl groups (which are generally different from one another) and X indicates the halide counter ion. It should be particularly noted that in this formula, the cation is a non-aromatic heterocyclic system. Specifically, N-methyl-N-ethyl pyrrolidinium bromide (abbreviated MEP) and N-methyl-N-ethyl morpholinium bromide (abbreviated MEM) are both commercially used for that purpose. However, the experimental results reported below indicate that neither N-methyl-N-ethyl pyrrolidinium bromide nor N-methyl-N-ethyl morpholinium bromide is suitable for use in hydrogen/ bromine regenerative fuel cells, for the reason that they crystallize under certain working conditions employed in such cells.

It has now been found that 1-alkyl-2-alkyl pyridinium bromide, such as 1-ethyl-2-methyl pyridinium bromide (abbreviated 2-MEPy), 1-alkyl-3-alkyl pyridinium bromide, such as 1-ethyl-3-methyl pyridinium bromide (abbreviated 3-MEPy), and 1,3 dialkyl imidazolium bromide such as 1-butyl 3-methyl imidazolium bromide (abbreviated BMIBr) and mixtures thereof are effective as complexing agents for a hydrogen/ bromine cells, e.g., a hydrogen/ bromine regenerative fuel cell. Having tested HBr/bromine containing electrolytes with varied compositions corresponding to distinct states ensuing during the charge/discharge cycle of hydrogen/ bromine cell, it has been surprisingly found that the presence of said compounds in the electrolyte allows the formation of polybromide complexes which do not solidify under the relevant working conditions.

Heretofore, 1-alkyl-2-methyl-pyridinium (also named N-alkyl picolinium) halide salts were proposed in the art for the following uses. US 5,260,148, for example, describes the preparation of an electrolytic solution for lithium secondary batteries by adding N-methyl picolinium ions to a solvent which is an equimolar mixture of propylene carbonate and 1,2-dimethoxyethane. Barlet, R. et al. [Journal de Chimie Physique et de Physico-Chimie Biologique (1984), 81(5), 349-54] describes the use of pyridinium halides as room temperature battery electrolytes. EP 0404188 discloses a non-aqueous electrolytic aluminum plating bath composition comprising, *inter alia*, halide such as an N-alkyl picolinium halide. Shlyapnikov, D.S. [Khimiya Geterotsiklicheskikh Soedinenii (1972), (7), 966-9] describes SO₂ complexes with quaternary halide salts of e.g. α-picoline.

The present invention is therefore primarily directed to the use of 1-alkyl-2-alkyl pyridinium halide, 1-alkyl-3-alkyl pyridinium halide, 1,3 dialkyl imidazolium halide or their mixtures, wherein the halide is preferably bromide, as bromine complexing agents in electrochemical flow cells selected from the group consisting of hydrogen/bromine cell. The alkyl groups attached to the aromatic ring are independently selected from the group of C1-C5 alkyl. Preferably, the alkyl groups are different from one another. In the case of 1-alkyl-2-alkyl pyridinium bromide and 1-alkyl-3-alkyl pyridinium bromide, it is preferred to have an ethyl group attached to the nitrogen atom and a methyl group attached to the carbon ring (i.e., either at the 2-or 3-position of the pyridine ring).

In another aspect, the invention provides an electrolyte suitable for use in electrochemical flow cells selected from the group consisting of hydrogen/bromine cell, said electrolyte comprising aqueous hydrogen bromide and a liquid complex composed of at least one of 1-alkyl-2-alkyl pyridinium halide (e.g., bromide), 1-alkyl-3-alkyl pyridinium halide (e.g., bromide) or 1,3 dialkyl imidazolium halide (e.g., bromide) combined with one or more bromine molecules. The liquid complex is preferably composed of 1-ethyl-2-methyl pyridinium bromide, 1-ethyl-3-methyl pyridinium bromide or 1-butyl 3-methyl imidazolium bromide and bromine molecules.

In another aspect, the invention is directed to a process for operating an electrochemical flow cell selected from the group consisting of hydrogen/bromine, comprising adding 1-alkyl-2-alkyl pyridinium halide (e.g., bromide), 1-alkyl-3-alkyl pyridinium halide (e.g., bromide), 1,3 dialkyl imidazolium halide (e.g., bromide) or their mixtures as set forth above, to the HBr-containing electrolyte of said cell.

The preferred complexing agents according to the invention, 1-ethyl-2-methyl pyridinium bromide and 1-ethyl-3-methyl pyridinium bromide are prepared by reacting 2-picoline or 3-picoline, respectively, with ethyl bromide, as illustrated by the following reaction schemes:

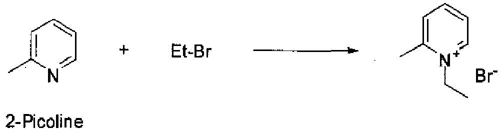

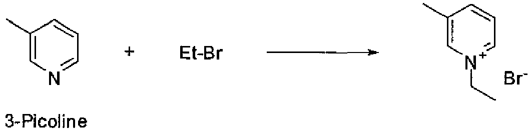

The reaction is carried out by charging a pressure reactor with the reactants and optionally also with a solvent, which may be either an aqueous or organic solvent. Alternatively, the reaction is solvent free, with one of the reactants being optionally used in excess. It is possible to introduce the entire amounts of the reactants into the reactor and then start the reaction by heating the reaction mixture. However, it is also possible to gradually feed one or more of the reactants (e.g., the ethyl bromide) into the reactor over a period of not less than one hour under heating.

The reaction mixture is heated, preferably to a temperature of not less than 90°C, and the reaction is allowed to proceed under pressure for a few hours. The product is conveniently collected in the form of an aqueous solution, which can be directly applied as an additive for the HBr electrolyte solution in accordance with the present invention. To this end, upon completion of the reaction, the organic solvent and/or residual amounts of the starting materials are removed from the reaction vessel by means of methods known in the art, e.g., distillation. Water can then be added into the reactor, to afford the complexing agent in an aqueous form. The concentration of the aqueous solution of 2-MEPy or 3-MEPY or their mixture which can be used as an additive for the HBr electrolyte is preferably from 50 to 90 wt%.

Another complexing agent suitable for use according to the invention, 1-butyl 3-methyl imidazolium bromide, is commercially available from Chemada Israel, and can be also prepared by methods known in the art.

The electrolyte according to the invention is prepared by combining together aqueous hydrogen bromide, the complexing agent, e.g., 1-ethyl-2-methyl pyridinium bromide, 1-ethyl-3-methyl pyridinium bromide, 1-butyl 3-methyl imidazolium bromide or a mixture thereof, and the electrochemically generated bromine, which is formed in-situ in the cell on charging, or chemically (e.g., peroxide) generated bromine. To an aqueous solution of hydrogen bromide, with HBr concentration of 5% - 52% by weight, e.g., 10-45 wt%, will be added the complexing agent such that its concentration in the resulting solution is not less than 0.25M, up to a concentration capable of complexing the maximal bromine content. On charging, the hydrogen bromide is consumed and bromine is generated. On discharging, the aqueous phase of the electrolyte is again concentrated with respect to HBr, and the concentration of bromine is reduced.

As noted above, the bromine complexing agents of the invention may be used either in individual form or in the form of mixtures, e.g., binary mixtures, in which the molar ratio between the two components of the mixture may be from 1:5 to 5:1, more preferably from 1:4 to 4:1 and even more preferably, from 1:3 to 3:1. One preferred mixture consists of 1-ethyl-2-methyl pyridinium bromide and 1-ethyl-3-methyl pyridinium bromide in a molar ratio between 1:2 to 1:4. The comlexing agents are preferably added to the electrolyte in the form of concentrated aqueous solutions in which the concentration of the complexing agent may be from 40 to 92% by weight, e.g., 65 to 90% by weight.

The process of the invention is carried out utilizing a hydrogen/bromine cell of the type described above with reference to Figure 1, with the addition of the complexing agent into the storage tank used for holding the aqueous HBr (indicated by numeral 5 in Figure 1). Another example of hydrogen/bromine cell which can be used in the process of the invention is illustrated in US 4,520,081. Vanadium/bromine cells are illustrated, for example, in US 7,320,844 or US 2006/0183016.

The following non-limiting working examples illustrate various aspects of the present invention.

### Examples

### Methods

1) The specific conductivity of the hydrogen bromide acid solutions containing the complexing agents were measured at room temperature, before the addition of bromine to the samples using Innolab 740 instrument with graphite conductivity cell.
2) The temperature at which the formation of a solid phase takes place in the electrolyte solution was determined by gradually cooling the samples from RT (approximately 25-30°C) to -15°C. The cooling regime was as follows: the temperature was decreased from RT down to 15°C with a cooling rate of 0.2°C/min, and kept at 15 °C for 4 hours and so forth down to -15°C. At each of the following temperatures: 15°C, 10°C, 5°C, 0°C, -5°C, -10°C and-15°C, the solution was maintained at a constant temperature for four hours. The cooling test was performed in polyethylene glycol solution, until the formation of crystals was observed.
3) The bromine concentration in the aqueous phase above the polybromide complex-oily phase was determined by a conventional iodometric titration technique. Each vial was sampled three times at room temperature.
4) The vapor pressure above the electrolyte solutions containing the complexing agents was measured at 20-26°C according to "Vapor pressures of bromine-quaternary ammonium salt complexes for zinc-bromine battery applications" Satya N. Bajpal J. Chem. Eng. Data 1981, 26, 2-4.

### Example 1

### Preparation of 2-MEPy in aqueous medium

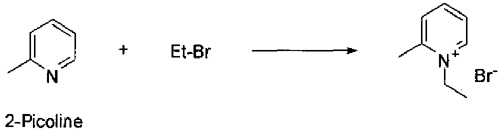

A pressure reactor was equipped with a mechanical stirrer with a magnetic relay and a thermocouple well. The reactor was purged with nitrogen, charged with 2-picoline (101.3 g) and de-ionized water (DIW) (20 mL), sealed and the mixture was heated to 92 °C. Ethyl bromide (97.9 g) was slowly added during 3 hours, at 92-100 °C. The mixture was heated at 94-100 °C for additional 2 hours, then cooled, and the pressure was released. The crude solution was diluted with DIW (24 mL) and excess 2-picoline was distilled-off as aqueous azeotrope, under reduced pressure. Finally, the residue was diluted with DIW. Final product: 251 g; 66.1 weight % (argentometric titration); yield, 91.5 %.

### Example 2

### Preparation of 2-MEPy in acetonitrile as a solvent

A pressure reactor was equipped with a mechanical stirrer with a magnetic relay and a thermocouple well. The reactor was purged with nitrogen, charged with 2-picoline (57.9 g), ethyl bromide (69 g) and acetonitrile (69 g). The reactor was sealed and the mixture heated to 97 °C. Heating at 97 °C was continued for 6 hours. Distillation of the solvent was controlled by the upper valve of the reactor followed by vacuum distillation (without cooling). DIW (31 mL) was added to dissolve the crude mixture and vacuum was applied to remove residual acetonitrile. Finally, the solution was diluted with DIW (10.5 g). Final product: 149 g; 80.0 weight % (argentometric titration); yield, 95 %.

### Example 3

### Preparation of 2-MEPy with excess ethyl bromide

A pressure reactor was equipped with a mechanical stirrer with a magnetic relay and a thermocouple well. The reactor was purged with nitrogen, charged with 2-picoline (95 g) and ethyl bromide (145 g). The reactor was sealed and the mixture heated to 97 °C. Heating at 97 °C was continued for 18 hours. Distillation of excess ethyl bromide was controlled by the upper valve of the reactor followed by vacuum distillation. Finally, the solution was diluted with DIW (47 g). Final product: 250 g; 79.3 weight % (argentometric titration); yield, 96 %.

### Example 4

### Preparation of 3-MEPy or 4-MEPy

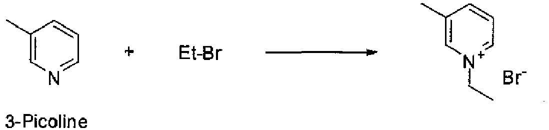

A pressure reactor was equipped with a mechanical stirrer with a magnetic relay and a thermocouple well. The reactor was purged with nitrogen, charged with 3-picoline (101.3 g) and DIW (25 mL). The reactor was sealed and the mixture was heated to 96 °C. Ethyl bromide (97.9 g) was slowly added during 2 hours, at 96-104 °C. The mixture was heated at 100 °C for additional 3.5 hours, after which time the pressure was released. The crude solution was diluted with DIW and excess 3-picoline was distilled-off as aqueous azeotrope, under reduced pressure. Finally, the residue was diluted with DIW. Final product: 260 g; 66.6 weight % (argentometric titration); yield, 95.6 %. 4-MEPy was prepared in a similar manner, starting from 4-picoline.

### Examples 5-7 (of the invention) and 8-10 (comparative)

### Preparing and measuring the properties of electrolyte solutions which correspond to electrolyte solutions at the beginning of the charge stage

Samples of hydrogen bromide acid solutions containing the complexing agents (abbreviated Quats), were prepared with final HBr concentration of 34% by weight, 0.8M of Quat and 0.2M of bromine, namely, with a composition corresponding to the composition of an electrolyte at the beginning of a charging process in hydrogen/bromine cell. The total volume of each sample was 12 ml in a closed vial. The samples were stored at room temperature (RT) for 24 hours after preparation before any measurement was conducted. The samples were tested for the following properties: the temperature at which a solid phase is formed in the electrolyte, free bromine concentration, conductivity and vapor pressure. The results are given in the following table:

**Table 1**

| Ex. | Quat | Temperature at which a solid phase was observed | [Br₂] in aqueous phase (%) | Specific conductivity (mS/cm) | Vapor pressure (mm Hg) |
|---|---|---|---|---|---|
| 5 | 2-MEPy | 5°C | 0.9 | 588 | 24 |
| 6 | 3-MEPy | -5°C | 0.65 | 623 | 24 |
| 7 | BMIBr | -10°C | 0.13 | 534 | 22 |
| 8 | 4-MEPy | 25°C | N/A | 610 | N/A |
| 9 | MEP | 20°C | N/A | 600 | N/A |
| 10 | MEM | 20°C | N/A | 597 | N/A |

The results show that 2-MEPy, 3-MEPy and BMIBr are suitable for use as bromine complexing agents in electrolyte solutions of hydrogen/ bromine cells at the beginning of the charge state.

### Example 11-13 (of the invention) and 14-16 (comparative)

### Preparing and measuring the properties of electrolyte solutions which correspond to electrolyte solutions at the end of the charge stage

The procedures and measurements as set forth in the previous examples were repeated. However, the amounts of HBr and elemental bromine were adjusted to form samples of electrolyte solutions that represent the composition of the electrolyte at the end of the charge process. Hence, samples were prepared with final HBr concentration of 22% by weight, 0.8M of Quat and 1M of bromine. The total volume of each sample was 12 ml in a closed vial. The samples were stored at room temperature for 24 hours after preparation before any measurement was conducted. The samples were tested for the following properties: the temperature at which a solid phase is formed in the electrolyte, free bromine concentration, conductivity and vapor pressure. The results are shown in Table 2.

**Table 2**

| Ex | Quat | solidification temperature | [Br₂] in aqueous phase (%) | Specific conductivity (mS/cm) | Vapor pressure (mm Hg) |
|---|---|---|---|---|---|
| 11 | 2-MEPy | -10°C | 1.05 | 582 | 21 |
| 12 | 3-MEPy | -10°C | 0.99 | 605 | 18 |
| 13 | BMIBr | -7°C. | 0.85 | 530 | 19 |
| 14 | 4-MEPy | 25°C | N/A | 597 | N/A |
| 15 | MEP | 5°C | 1.21 | 593 | 24 |
| 16 | MEM | 5°C | 2.14 | 591 | 30 |

The results show that 2-MEPy, 3-MEPy and BMIBr are suitable for use as bromine complexing agents in electrolyte solutions of hydrogen/ bromine cells at the end of the charge stage.

### Examples 17-21 (of the invention) and 22-23 (comparative)

In this set of examples, various mixtures of complexing agents were tested in electrolyte solutions having a composition which corresponds to the composition of an electrolyte solution at the beginning of the charge process (see the procedure of Examples 5-10). The results are tabulated in Table 3.

**Table 3**

| Ex | Quats mixture | Quats ratio | solidification temperature | [Br₂] in aqueous phase (%) | Specific conductivity (mS/cm) | Vapor pressure (mm Hg) |
|---|---|---|---|---|---|---|
| 17 | 2-MEPy/ 3-MEPy | 3:1 | -5°C | 0.93 | 561 | 22 |
| 18 | 2-MEPy/ 3-MEPy | 1:1 | -10°C | 0.81 | 456 | 35 |
| 19 | 2-MEPy/ 3-MEPy | 1:3 | -10°C | 0.49 | 600 | 36 |
| 20 | 2-MEPy/ 3-MEPy/ 4-MEPy | 1:1:1 | -10°C | 1.38 | 598 | - |
| 21 | 2-MEPy/ 4-MEPy | 3:1 | -5°C | 0.8 | 572 | - |
| 22 | 2-MEPy/ 4-MEPy | 1:1 | 20°C | N/A | N/A | N/A |
| 23 | 2-MEPy/ 4-MEPy | 1:3 | 20°C | N/A | N/A | N/A |

### Examples 24-29 (of the invention) and 30 (comparative)

In this set of examples, various mixtures of complexing agents were tested in electrolyte solutions having a composition which corresponds to the composition of an electrolyte solution at the end of the charge process (see the procedure of Examples 11-16). The results are tabulated in Table 4.

**Table 4**

| Ex | Quats mixture | Quats ratio | solidification temperature | [Br₂] in aqueous phase (%) | Specific conductivity (mS/cm) | Vapor pressure (mm Hg) |
|---|---|---|---|---|---|---|
| 24 | 2-MEPy/ 3-MEPy | 3:1 | -10°C | 1.12 | 569 | 27 |
| 25 | 2-MEPy/ 3-MEPy | 1:1 | -1D°C | 0.88 | 562 | 34 |
| 26 | 2-MEPy/ 3-MEPy | 1:3 | -10°C | 1.16 | 566 | 38 |
| 27 | 2-MEPy/ 3-MEPy/ 4-MEPy | 1:1:1 | -10°C | 1.12 | 576 | - |
| 28 | 2-MEPy/ | 3:1 | -10°C | 0.92 | 571 | 43 |
| 29 | 2-MEPy/ 4-MEPy | 1:1 | -5°C | 1.25 | - | - |
| 30 | 2-MEPy/ 4-MEPy | 1:3 | 20°C | - | - | - |

### Examples 31-36

Samples were prepared with HBr concentration of 10% by weight. The complexing agent that was tested was 2-MEPy. The concentration of 2-MEPy in each sample was 0.8M. Different amounts of elemental bromine were added to the samples and some properties of interest (the concentration of elemental bromine in the aqueous phase, the conductivity and vapor pressure) were measured at two temperatures: 22°C and 45°C.

The following is noted with respect to the measurements relevant to this set of examples (31-36) and the next two sets of examples (37-42 and 43-48):
The samples were stored at 25°C for at least 24 hours after preparation before any measurement was conducted.

Conductivity measurements were carried out at 22-24°C on solutions which contain bromine.
Sample preparations for iodometric titration and the titration itself were done at 22-24°C.
The equilibrium total pressure above the electrolyte at the desired temperature has been measured using mercury manometer vs equilibrium pressure of liquid for which exact values of equilibrium vapor pressure are well known in all range of temperatures. Distillated water was used as the reference. Two round-bottom flasks of the same volume and with the same volume of the measured electrolyte and water, closed by vacuum valves, were connected to the mercury manometer. Each flask was accurately equilibrated at the desired temperature and the vacuum valves were opened. After the system was equilibrated the difference between the levels of mercury in both side of manometer tube was measured. The accurate value of water pressure at temperature of water flask is known. The measured difference in mercury levels has been added to this value.

The results are tabulated in Table 5.

**Table 5**

| Ex. | Br₂, M added | [Br₂] in aqueous phase (%) | Conductivity, mS/cm | Vapor pressure at 22°C, mmHg | Vapor pressure at 45°C, mmHg |
|---|---|---|---|---|---|
| 31 | - | - | 352 | 17 | 78 |
| 32 | 1.0 | 0.42 | 479 | - | - |
| 33 | 1.5 | 0.79 | 480 | - | - |
| 34 | 2 | 1.92 | 485 | 10 | 63 |
| 35 | 2.5 | 3.72 | 481 | 14 | 77 |
| 36 | 3 | 6.07 | 479 | 18 | 82 |

### Examples 37-42

Samples were prepared with HBr concentration of 10% by weight. The complexing agent that was tested was a mixture consisting of 2-MEPy and 3-MEPy at molar ratio of 3:1. The concentration of the mixture of 2-MEPy and 3-MEPy in each sample was 0.8M. Different amounts of elemental bromine were added to the samples and some properties of interest (the concentration of elemental bromine in the aqueous phase, the conductivity and the vapor pressure) were measured at three temperatures: 22°C, 45°C and 60°C. The results are tabulated in Table 6.

**Table 6**

| Ex. | Br₂, M added | [Br₂] in aq. phase (%) 22°C | [Br₂] in aq. phase (%) 45°C | Cond. mS/cm | Vapor pressure at 22°C, ±1 mmHg | Vapor pressure at 45°C, ±2 mmHg | Vapor pressure at 60°C, ±3 mmHg |
|---|---|---|---|---|---|---|---|
| 37 | - | | | 365 | 15 | 80 | 165 |
| 38 | 1.0 | 0.64 | 0.75 | 460 | - | - | - |
| 39 | 1.5 | 0.95 | 1.20 | 478 | - | - | - |
| 40 | 2 | 1.17 | 2.73 | 475 | - | - | - |
| 41 | 2.5 | 2.79 | 5.31 | 467 | 16 | 75 | 176 |
| 42 | 3 | 5.16 | 8.76 | 462 | 17 | 77 | 185 |

### Examples 43-48

Samples were prepared with HBr concentration of 10% by weight. The complexing agent that was tested was a mixture consisting of 2-MEPy and 3-MEPy at molar ratio of 1:3. The concentration of the mixture of 2-MEPy and 3-MEPy in each sample was 0.8M. Different amounts of elemental bromine were added to the samples and some properties of interest (the concentration of elemental bromine in the aqueous phase, the conductivity and the vapor pressure) were measured at three temperatures: 22°C, 45°C and 60°C. The results are tabulated in Table 7.

**Table 7**

| Ex. | Br₂, M added | [Br₂] in aq. phase (%) 22°C | [Br₂] in aq. phase (%) 45°C | Cond. mS/cm | Vapor pressure at 22°C, mmHg | Vapor pressure at 45°C, mmHg | Vapor pressure at 60°C, mmHg |
|---|---|---|---|---|---|---|---|
| 43 | - | | | 380 | 17 | 74 | 158 |
| 44 | 1.0 | 0.69 | 1.90 | 463 | - | - | - |
| 45 | 1.5 | 0.94 | 1.79 | 472 | - | - | - |
| 46 | 2 | 1.40 | 2.15 | 475 | - | - | - |
| 47 | 2.5 | 2.82 | 5.56 | 465 | 17 | 67 | 170 |
| 48 | 3 | 6.66 | 8.36 | 466 | 17 | 70 | 183 |

It is apparent from Tables 5, 6 and 7 that at temperatures of 22°C and 45°C, the increase at the amount of elemental bromine in the electrolyte does not result in an increase of the vapor pressure, indicating that the additives of the invention form strong complexes with the elemental bromine in HBr solutions. It should be noted that at a temperature of 60°C a small increase of the vapor pressure is observed, but this temperature is beyond the temperature range at which electrochemical cells normally operate.

## Claims

1. An electrolyte suitable for use in an electrochemical flow cell, said electrolyte comprising aqueous hydrogen bromide and a liquid complex composed of at least one of 1-alkyl-2-alkyl pyridinium halide, 1-alkyl-3-alkyl pyridinium halide or 1-alkyl-3-alkyl imidazolium halide combined with one or more bromine molecules.

2. An electrolyte according to claim 1, comprising a liquid complex composed of at least one of 1-ethyl-2-methyl pyridinium bromide, 1-ethyl-3-methyl pyridinium bromide or 1-butyl 3-methyl imidazolium bromide combined with one or more bromine molecules.

3. An electrolyte according to claim 2, comprising a liquid complex composed of at least one of 1-ethyl-2-methyl pyridinium bromide or 1-ethyl-3-methyl pyridinium bromide, combined with one or more bromine molecules.

4. An electrolyte according to claim 3, comprising a mixture of 1-ethyl-2-methyl pyridinium bromide and 1-ethyl-3-methyl pyridinium bromide.

5. Use of at least one of 1-alkyl-2-alkyl pyridinium halide, 1-alkyl-3-alkyl pyridinium halide or 1-alkyl-3-alkyl imidazolium halide as bromine-complexing agents in an electrochemical flow cell which contains an electrolyte comprising aqueous hydrogen bromide, said cell being hydrogen/bromine cell.

6. Use according to claim 5, wherein the 1-alkyl-2-alkyl pyridinium halide is 1-ethyl-2-methyl pyridinium bromide.

7. Use according to claim 5, wherein the 1-alkyl-3-alkyl pyridinium halide is 1-ethyl-3-methyl pyridinium bromide.

8. Use according to claim 5, wherein a mixture of 1-ethyl-2-methyl pyridinium bromide and 1-ethyl-3-methyl pyridinium bromide is used.

9. A process for operating an electrochemical flow cell which is a hydrogen/bromine cell, comprising adding 1-alkyl-2-alkyl pyridinium halide, 1-alkyl-3-alkyl pyridinium halide, 1-alkyl-3-alkyl imidazolium halide or their mixture to the HBr-containing electrolyte of said cell.

10. A process according to claim 9, comprising adding 1-ethyl-2-methyl pyridinium bromide, 1-ethyl-3-methyl pyridinium bromide or a mixture thereof to the HBr-containing electrolyte in hydrogen/bromine cell.

## Patentansprüche

1. Elektrolyt, der für die Verwendung in einer elektrochemischen Durchflusszelle geeignet ist, wobei der Elektrolyt wässrigen Bromwasserstoff und einen flüssigen Komplex, der aus mindestens einem von 1-Alkyl-2-alkylpyridiniumhalogenid, 1-Alkyl-3-alkylpyridiniumhalogenid oder 1-Alkyl-3-alkylimidazoliumhalogenid gemeinsam mit einem oder mehreren Brommolekülen zusammengesetzt ist, umfasst.

2. Elektrolyt gemäß Anspruch 1 umfassend einen flüssigen Komplex, der aus mindestens einem von 1-Ethyl-2-methylpyridiniumbromid, 1-Ethyl-3-methylpyridiniumbromid oder 1-Butyl-3-methylimidazoliumbromid gemeinsam mit einem oder mehreren Brommolekülen zusammengesetzt ist.

3. Elektrolyt gemäß Anspruch 2 umfassend einen flüssigen Komplex, der aus mindestens einem von 1-Ethyl-2-methylpyridiniumbromid oder 1-Ethyl-3-methylpyridiniumbromid gemeinsam mit einem oder mehreren Brommolekülen zusammengesetzt ist.

4. Elektrolyt gemäß Anspruch 3 umfassend eine Mischung aus 1-Ethyl-2-methylpyridiniumbromid und 1-Ethyl-3-methylpyridiniumbromid.

5. Verwendung von mindestens einem aus 1-Alkyl-2-alkylpyridiniumhalogenid, 1-Alkyl-3-alkylpyridiniumhalogenid oder 1-Alkyl-3-alkylimidazoliumhalogenid als Brom-Komplexbildner in einer elektrochemischen Durchflusszelle, welche einen Elektrolyten, der wässrigen Bromwasserstoff umfasst, enthält, wobei die Zelle eine Wasserstoff/Brom-Zelle ist.

6. Verwendung gemäß Anspruch 5, wobei das 1-Alkyl-2-alkylpyridiniumhalogenid 1 -Ethyl-2-methlpyridiniubromid ist.

7. Verwendung gemäß Anspruch 5, wobei das 1-Alkyl-3-alkylpyridiniumhalogenid 1-Ethyl-3-methlpyridiniubromid ist.

8. Verwendung gemäß Anspruch 5, wobei eine Mischung aus 1-Ethyl-2-methylpyridiniumbromid und 1-Ethyl-3-methylpyridiniumbromid verwendet wird.

9. Verfahren zum Betreiben einer elektrochemischen Durchflusszelle, welche eine Wasserstoff/Brom-Zelle ist, umfassend das Zugeben von 1-Alkyl-2-alkylpyridiniumhalogenid, 1-Alkyl-3-alkylpyridiniumhalogenid, 1-Alkyl-3-alkylimidazoliumhalogenid oder ihrer Mischung zu dem HBr-haltigen Elektrolyten von der Zelle.

10. Verfahren gemäß Anspruch 9 umfassend das Zugeben von 1-Ethyl-2-methylpyridiniumbromid, 1-Ethyl-3-methylpyridiniumbromid oder einer Mischung davon zu dem HBr-haltigen Elektrolyten in der Wasserstoff/Brom-Zelle.

## Revendications

1. Un électrolyte adapté pour l'utilisation dans une cellule de débit électrochimique, ledit électrolyte comprenant une solution aqueuse de bromure d'hydrogène et un complexe liquide composé d'au moins un d'halogénure de 1-alkyl-2-alkyl-pyridinium, d'halogénure de 1-alkyl-3-alkyl-pyridinium ou d'halogénure de 1-alkyl-3-alkyl-imidazolium, combiné avec une ou plusieurs molécules de brome.

2. Un électrolyte selon la revendication 1, comprenant un complexe liquide composé d'au moins un de bromure de 1-ethyl-2-methyl-pyridinium, de bromure de 1-ethyl-3-methyl-pyridinium ou de bromure de 1-butyl-3-methyl-imidazolium, combiné avec une ou plusieurs molécules de brome.

3. Un électrolyte selon la revendication 2, comprenant un complexe liquide composé d'au moins un de bromure de 1-ethyl-2-methyl-pyridinium ou de bromure de 1-ethyl-3-methyl-pyridinium, combiné avec une ou plusieurs molécules de brome.

4. Un électrolyte selon la revendication 3, comprenant un mélange de bromure de 1-ethyl-2-methyl-pyridinium et de bromure de 1-ethyl-3-methyl-pyridinium.

5. Utilisation d'au moins un d'halogénure de 1-alkyl-2-alkyl-pyridinium, d'halogénure de 1-alkyl-3-alkyl-pyridinium ou d'halogénure de 1-alkyl-3-alkyl-imidazolium en tant qu'agent complexant du brome dans une cellule de débit électrochimique qui contient un électrolyte comprenant une solution aqueuse de bromure d'hydrogène, ladite cellule étant une cellule hydrogène/brome.

6. Utilisation selon la revendication 5, dans laquelle l'halogénure de 1-alkyl-2-alkyl-pyridinium est du bromure de 1-ethyl-2-methyl-pyridinium.

7. Utilisation selon la revendication 5, dans laquelle l'halogénure de 1-alkyl-3-alkyl-pyridinium est du bromure de 1-ethyl-3-methyl-pyridinium.

8. Utilisation selon la revendication 5, dans laquelle un mélange de bromure de 1-ethyl-2-methyl-pyridinium et de bromure de 1-ethyl-3-methyl-pyridinium est utilisé.

9. Procédé pour l'opération d'une cellule de débit électrochimique qui est une cellule hydrogène/brome, comprenant l'addition d'halogénure de 1-alkyl-2-alkyl-pyridinium, d'halogénure de 1-alkyl-3-alkyl-pyridinium, d'halogénure de 1-alkyl-3-alkyl-imidazolium, ou de leur mélange a l'électrolyte contenant de la HBr de ladite cellule.

10. Un procédé selon la revendication 9, comprenant l'addition de bromure de 1-ethyl-2-methyl-pyridinium, de bromure de 1-ethyl-3-methyl-pyridinium, ou de leur mélange a l'électrolyte contenant de la HBr de ladite cellule.
